# EUROPEAN PATENT SPECIFICATION

(11) **EP 3 478 200 B1**
(45) Date of publication and mention of the grant of the patent: **04.12.2019**
(21) Application number: 17739495.4
(22) Date of filing: 27.06.2017
(51) Int. Cl.: A61B 18/20, B26B 19/38

(54) **A GUARD STRUCTURE FOR A HAIR CUTTING DEVICE**
SCHUTZSTRUKTUR FÜR EINE HAARSCHNEIDEVORRICHTUNG
STRUCTURE DE PROTECTION POUR UN DISPOSITIF DE COUPE DE CHEVEUX

(30) Priority: 30.06.2016 EP 16177325
(43) Date of publication of application: 08.05.2019
(73) Proprietor: Koninklijke Philips N.V., 5656 AE Eindhoven (NL)
(72) Inventor: BOURQUIN, Yannyk, Parulian, Julian, 5656 AE Eindhoven (NL); MOESKOPS, Bastiaan, Wilhelmus, Maria, 5656 AE Eindhoven (NL); THUMMA, Kiran, Kumar, 5656 AE Eindhoven (NL); JOHNSON, Mark, Thomas, 5656 AE Eindhoven (NL); VERHAGEN, Rieko, 5656 AE Eindhoven (NL)
(74) Representative: Uittenbroek, Arie Leendert
(86) International application number: PCT/EP2017/065775
(87) International publication number: WO 2018/002000

(56) References cited:
- EP-A1- 2 656 982
- WO-A1-2014/143670
- US-A1- 2008 244 912

## Description

### FIELD OF THE INVENTION

The invention relates to a hair cutting device for cutting (e.g. shaving) hair on a body of a subject, and in particular relates to a guard structure for a hair cutting device that uses laser light to cut or shave hair, and a hair cutting device comprising the same.

### BACKGROUND OF THE INVENTION

Shaving devices for cutting or shaving hair on a body of a subject typically make use of one or more blades that cut hairs as the blade is moved across the skin of the subject. The blades can be static within the device, for example as in a wet razor, whereas in other types of devices, for example electric shavers, one or more blade elements can be actuated (e.g. rotated or oscillated) in order to produce a cutting action.

However, an alternative type of shaving device has been proposed in WO 2014/143670 that makes use of laser light. In particular a laser light source is provided that is configured to generate laser light having a wavelength selected to target a predetermined chromophore to effectively cut a hair shaft. A fiber optic is located on a shaving portion of the device that is positioned to receive the laser light from the laser light source at a proximal end, conduct the laser light from the proximal end toward a distal end, and emit the light out of a cutting region of the fiber optic and toward hair when the cutting region is brought in contact with the hair.

It is to be noted that US 2008/244912 A1 discloses a further alternative type of shaving device that makes use of light. In particular laser light is couple out of an array of optical components which are aligned with an optic such as a blade. In this configuration the optic is to manipulate and direct the light to cut hair shafts.

### SUMMARY OF THE INVENTION

To achieve good shaving closeness, the cutting element of the shaving device (i.e. the fiber optic in the case of the device in WO 2014/143670) needs to be brought very close to the skin or even touch the skin. That device is made in such way that the light couples into a hair when a hair is in contact with the fiber optic. In particular, this is achieved by the core of the fiber optic having a lower refractive index than hair. However, since the refractive index of skin is close to the refractive index of hair, the laser light will also be able to couple into the skin if the cutting element is brought into contact with the skin. This will potentially lead to burning or irritation of the skin and create a significant safety issue for this type of shaving device.

Thus, to reduce the risk of damage or injury to the skin of the subject from optical energy, it is proposed herein to provide a guard structure to space an optical waveguide cutting element from the skin so that laser light can couple into hair when hair is in contact with the optical waveguide, and reduce the amount of light that couples into the skin. The use of a guard structure to space the optical waveguide cutting element from the skin is possible since an optical waveguide-based hair cutting device does not require the skin to act as a counter cutting surface in order to achieve the cutting action.

However, as the guard structure is in contact with the optical waveguide, it is possible for light in the optical waveguide to couple into the guard structure, which can lead to a loss of light energy/power for cutting hairs and which can lead to the guard structure being heated, which can cause irritation or damage to the skin of the subject.

Therefore there is a need for a guard structure for use in a hair cutting device that reduces the risk of damage or injury to the skin of the subject from optical energy.

According to a first aspect, there is provided a guard structure for use in a hair cutting device for cutting hair on a body of a subject, the guard structure comprising one or more guard elements, wherein the one or more guard elements are arranged such that in use in the hair cutting device the one or more guard elements space an optical waveguide in the hair cutting device from the skin of the subject, and wherein the one or more guard elements are shaped and/or formed to minimise or reduce the coupling of light from the optical waveguide to the guard structure.

In some embodiments, the one or more guard elements are formed from a material having a lower refractive index than the optical waveguide. This helps to avoid light coupling into the one or more guard elements from the optical waveguide, and thus reduces the heating of the guard elements and maintains the light power available to cut hair.

In some embodiments, the one or more guard elements are formed with a layer or coating of material that has a lower refractive index than the optical waveguide. This helps to avoid light coupling into the one or more guard elements from the optical waveguide, and thus reduces the heating of the guard elements and maintains the light power available to cut hair.

In some embodiments, the one or more guard elements are shaped to reduce or minimise the contact area between the guard elements and the optical waveguide. This helps to reduce the amount of light that can couple into the one or more guard elements from the optical waveguide, and thus reduce the heating of the guard elements and maintain the light power available to cut hair.

In some embodiments, a side of each of said one or more guard elements that is in contact with the optical waveguide is curved. This curved side or surface helps to reduce the amount of light that can couple into the one or more guard elements from the optical waveguide, and thus reduce the heating of the guard elements and maintain the light power available to cut hair.

In some embodiments, a side of each of said one or more guard elements that is in contact with the optical waveguide comprises one or more protrusions that provide the contact between the one or more guard elements and the optical waveguide. These protrusions help to reduce the amount of light that can couple into the one or more guard elements from the optical waveguide, and thus reduce the heating of the guard elements and maintain the light power available to cut hair.

In some embodiments, the one or more guard elements comprise a coating or layer of an optically reflective material on at least a side of the one or more guard elements that contact the optical waveguide. This helps to reduce the amount of light that can couple into the one or more guard elements from the optical waveguide by reflecting the light back towards or into the optical waveguide, and thus reduce the heating of the guard elements and maintain the light power available to cut hair.

In some embodiments, the one or more guard elements are coated with or comprise a hydrophobic and/or oleophobic layer. This coating or layer helps to prevent or reduce the build-up of fluid between the guard elements, and between the guard elements and the optical waveguide, thereby maintaining the cutting efficiency of the optical waveguide.

In some embodiments, the one or more guard elements are elongate elements.

In alternative embodiments, the one or more guard elements is a grid or foil.

According to a second aspect, there is provided a hair cutting device for cutting hair on a body of a subject, the hair cutting device comprising a light source for generating laser light at one or more specific wavelengths corresponding to wavelengths absorbed by one or more chromophores in hair; a cutting element that comprises an optical waveguide that is coupled to the light source to receive laser light, wherein a portion of a sidewall of the optical waveguide forms a cutting face for contacting hair; and a guard structure as described above.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of a hair cutting device according to an embodiment of the invention;
Fig. 2 is a pair of schematic drawings showing different views of an exemplary hair cutting device according to an embodiment of the invention;
Fig. 3 is a graph illustrating the refractive index of hair;
Fig. 4 is an illustration of a guard structure according to an embodiment and an optical waveguide cutting element;
Fig. 5 is an illustration of the guard structure according to a first specific embodiment and an optical waveguide cutting element;
Fig. 6 is an illustration of a guard structure according to a second specific embodiment and an optical waveguide cutting element;
Fig. 7 is an illustration of a guard structure according to a third specific embodiment and an optical waveguide cutting element;
Fig. 8 is an illustration of a guard structure according to a fourth specific embodiment and an optical waveguide cutting element; and
Fig. 9 is an illustration of a guard structure according to a fifth specific embodiment and an optical waveguide cutting element.

### DETAILED DESCRIPTION OF THE EMBODIMENTS

As noted above, the present invention provides an improvement in the safety and comfort of a subject that is using a laser light-based shaving device, for example as described in WO 2014/143670. In particular, it has been recognised that by providing a guard structure having one or more guard elements to space the optical waveguide from the skin, laser light can couple into hair when hair is in contact with the optical waveguide, but light will not be able to (or be less likely to) couple into the skin, thereby reducing the risk of burning or irritating the skin of the subject.

It will be appreciated that the invention is applicable to shaving devices (e.g. razors or electric shavers), and any other type of device that is used to cut hair (e.g. hair clippers), even if those devices do not necessary aim to provide a 'clean shave' (i.e. to remove hair at the level of the skin).

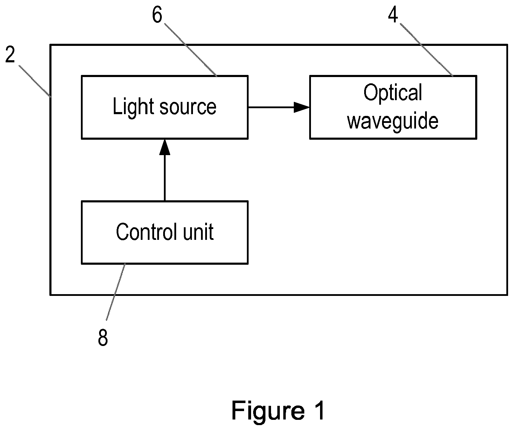

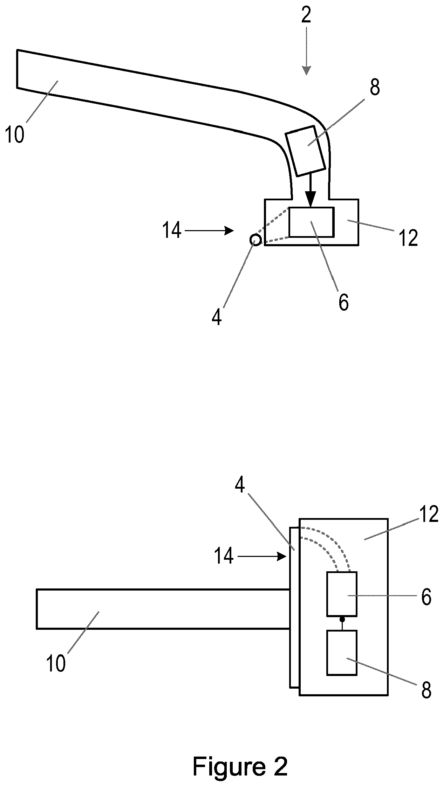

Fig. 1 is a block diagram of a hair cutting device 2 according to an embodiment of the invention. Fig. 2 shows a hair cutting device 2 in the form of a handheld razor according to an exemplary embodiment of the invention. The hair cutting device 2 is for cutting (e.g. shaving) hair on a body of a subject. The subject may be a person or an animal. The hair may be facial hair (i.e. hair on the subject's face), or hair on the subject's head or other part of their body (legs, chest, etc.).

The hair cutting device 2 comprises a cutting element 4 that enables hair to be cut as the hair cutting device 2 is moved over the skin of a subject. The cutting element 4 is an optical waveguide 4 that is arranged on the hair cutting device 2 so that the optical axis of the optical waveguide 4 (i.e. the line along which light typically propagates through the optical waveguide 4) is generally perpendicular to the direction in which the hair cutting device 2 is moved so that hairs contact the side wall of the optical waveguide 4 (the side wall corresponding to the long edge of the optical waveguide 4) as the hair cutting device 2 is moved across the skin of the subject. In some embodiments, the optical waveguide 4 is an optical fibre, although those skilled in the art will be aware of other types of optical waveguide that can be used according to the invention, such as a slab waveguide, a strip waveguide or a photonic crystal waveguide. An optical fibre comprises a core, and in some embodiments also comprises a cladding, which may or may not fully encompass the core (e.g. part of the core may be exposed).

A light source 6 is provided in the hair cutting device 2 that generates laser light at one or more specific wavelengths. The light source 6 is optically coupled to the optical waveguide 4 so that the laser light generated by the light source 6 is coupled into the optical waveguide 4 (and specifically coupled into an end of the optical waveguide 4 so that the laser light propagates through the optical waveguide 4).

The light source 6 is configured to generate laser light at one or more specific wavelengths that can be used to cut or burn through hair. In particular, each wavelength corresponds to the wavelength of light absorbed by a chromophore that is found in hair. As is known, a chromophore is the part of a molecule that provides the molecule with its colour. Thus, the laser light will be absorbed by the chromophore and converted into heat which will melt or burn the hair or otherwise destroy the bonds in the molecules of the hair, and it is this melting or burning that provides the cutting action of the hair cutting device 2.

Suitable chromophores that can be targeted by the laser light generated by the light source 6 include, but are not limited to, melanin, keratin and water. Suitable wavelengths of laser light that can be used include, but are not limited to, wavelengths selected from the range 380 nm (nanometers) to 500 nm and 2500 nm to 3500 nm. Those skilled in the art will be aware of the wavelengths of light that are absorbed by these chromophores, and thus also the specific wavelengths of light that the light source 6 should generate for this purpose, and further details are not provided herein.

In some embodiments the light source 6 can be configured to generate laser light at a plurality of wavelengths (either simultaneously or sequentially), with each wavelength being selected to target a different type of chromophore. This can improve the cutting action of the optical waveguide 4 since multiple types of molecules in the hair may be burnt using the laser light. Alternatively multiple light sources 6 can be provided that each generate laser light at a respective wavelength, and each light source 6 can be coupled to a respective optical waveguide 4 to provide multiple cutting elements 4 in the device 2.

The hair cutting device 2 also comprises a control unit 8 that controls the operation of the hair cutting device 2, and in particular is connected to the light source 6 to control the activation and deactivation of the light source 6 (and in some embodiments control the wavelength and/or intensity of the light generated by the light source 6). The control unit 8 may activate and deactivate the light source 6 in response to an input from a user of the hair cutting device 2. The control unit 8 can comprise one or more processors, processing units, multi-core processors or modules that are configured or programmed to control the hair cutting device 2.

As noted above, Fig. 2 shows a hair cutting device 2 that is in the form of a handheld wet razor. Fig. 2 shows a side view and a bottom view of the razor 2. The razor 2 comprises a handle 10 for the subject (or other user of the device 2) to hold, and a head portion 12 that includes the cutting element 4 (optical waveguide/fibre). As shown, the optical waveguide 4 is arranged along an edge of the head portion, and a part of the optical waveguide 4 forms (or corresponds to) a cutting face 14. The cutting face 14 is the part of the optical waveguide 4 that is intended to come into contact with hair as the hair cutting device 2 is moved across the skin of the subject. A light source 6 and control unit 8 are shown as being incorporated into the head portion 12 and handle 10 respectively, but it will be appreciated that the positions of these components in the hair cutting device 2 as shown in Fig. 2 is not limiting. Likewise it will be appreciated that the embodiment shown in Fig. 2 is merely an example, and the invention can be incorporated or used in any type of hair cutting device 2 that comprises an optical waveguide cutting element 4 as described herein.

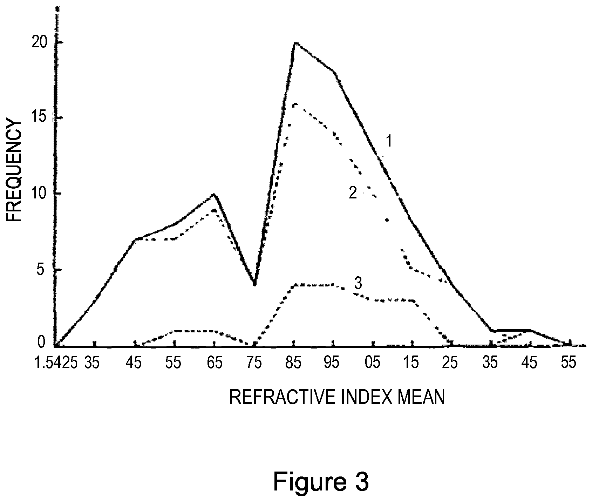

The graph in Fig. 3 illustrates the refractive index of hair, which can be found in a paper by M. D. Greenwell, A. Willner, Paul L. Kirk: Human Hair Studies: III. Refractive Index of Crown Hair, 31 Am. Inst. Crim. L. & Criminology 746 (1940-1941). Curve 1 is a composite line, curve 2 is a line representing the refractive index for Caucasian people, and curve 3 is a line representing the refractive index for non-Caucasian people. Thus, it can be seen that the refractive index of hair is between (approximately) 1.545 and 1.555, although there will be variation between individuals. For example the above paper also recognises that the refractive index of hair can depend on the sex of the subject, e.g. the refractive index of hair on a female is generally higher than the refractive index of hair on a male.

As is known, the optical waveguide 4 acts as a waveguide for the light coupled from the light source 6 through the occurrence of total internal reflection, since the refractive index of air is lower than that of the optical waveguide 4. However, if an object that has a refractive index higher than the optical waveguide 4 is put into contact with the optical waveguide 4, then the total internal reflection is 'frustrated' and light can couple from the optical waveguide 4 into that object. Thus, in order for light to be coupled into a hair from the optical waveguide 4 (to provide the cutting action according to the invention), the optical waveguide 4 must have the same or a lower refractive index than hair at the point at which the hair contacts the optical waveguide 4. Thus, the optical waveguide 4 must have the same or a lower refractive index than hair at least at the cutting face 14 portion of the optical waveguide 4. Preferably the refractive index of the optical waveguide 4 at the cutting face 14 is the same as that of hair since that provides the best coupling of light from the optical waveguide 4 to the hair.

Thus, in some embodiments, the refractive index of the optical waveguide 4 at least at the cutting face 14 is equal to or lower than 1.56. More preferably the refractive index of the optical waveguide 4 at least at the cutting face 14 is equal to or lower than 1.55. Even more preferably, the refractive index of the optical waveguide 14 at least at the cutting face 14 is equal to or lower than 1.54, since this refractive index is below the refractive indices identified in Fig. 3.

In some embodiments, a lower bound for the refractive index of the optical waveguide 4 at the cutting face 14 can be 1.48, 1.51, 1.53 or 1.54.

A range of values from which the refractive index of the optical waveguide 4 is selected can be formed from any combination of the upper and lower refractive index bounds set out in the preceding paragraphs.

The optical waveguide/fibre 4 can be made from any suitable material or combination of materials. For example optical waveguides/fibres can be composed of or comprise silica, fluoride glass, phosphate glass, chalcogenide glass, and/or crown glass (such as BK7).

As noted above, to achieve good shaving closeness, the optical waveguide 4 needs to be brought very close to the skin or even touch the skin. However, since the refractive index of skin is close to the refractive index of hair, the laser light will also be able to couple into the skin if the cutting element is brought into contact with the skin. This will potentially lead to burning or irritation of the skin. Thus, according to the invention a guard structure is provided that has one or more guard elements that space the optical waveguide 4 from the skin so that laser light can couple into hair when hair is in contact with the optical waveguide 4, but light will not be able to (or be less likely to) couple into the skin, thereby reducing the risk of burning or irritating the skin of the subject. In other words, the guard structure acts to reduce the size of the contact surface between the skin and the optical waveguide 4, which reduces the coupling of light into the skin.

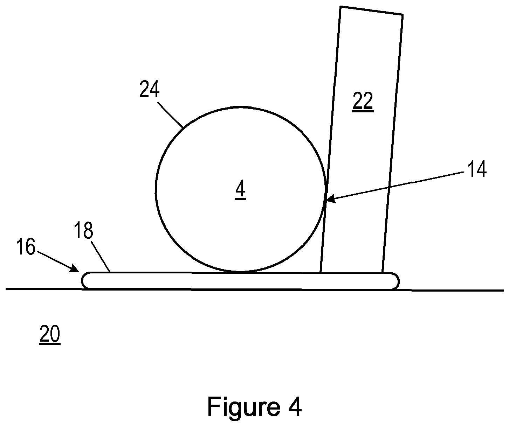

Fig. 4 illustrates an exemplary embodiment of the guard structure 16 according to the invention. The guard structure 16 is for use in or with a hair cutting device 2, for example as shown in Figs. 1 and 2. Fig. 4 shows the guard structure 16 as part of a hair cutting device 2, but only the optical waveguide 4 part of the hair cutting device 2 is shown. In Fig. 4 the guard structure 16 and the optical waveguide 4 are shown side on (i.e. looking down the optical axis of the optical waveguide 4), and no other support element for the optical waveguide 4 is shown. The guard structure 16 is also referred to as a 'hair skin manipulator' (HSM). The guard structure 16, and in particular the one or more guard elements 18, can be formed from or comprise any one or more of metal, glass, ceramic, plastic, crystal, composites, Teflon or oxides.

The guard structure 16 comprises one or more guard elements 18 that are arranged to space the optical waveguide 4 from the skin 20 of the subject. That is, the one or more guard elements 18 are arranged so that when the hair cutting device 2 is in use, the one or more guard elements 18 are between the optical waveguide 4 and the skin 20. The one or more guard elements 18 are shaped and arranged to allow hair 22 on the skin 20 to reach and contact the optical waveguide 4 as the hair cutting device 2 is moved over the skin 20 of the subject. Thus, spaces or gaps are provided between each pair of neighbouring guard elements 18 to allow hairs 22 to contact the optical waveguide 4.

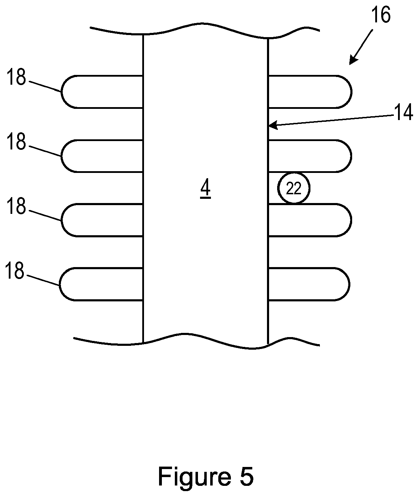

Fig. 5 is a top view of a guard structure 16 according to a first specific embodiment (i.e. a view onto the hair cutting device 2 when it is in contact with the skin). In the embodiment illustrated in Fig. 5, the guard structure 16 comprises a plurality of guard elements 18 in the form of elongate elements that are arranged with the guard elements 18 generally aligned with (i.e. generally parallel to) the direction in which the hair cutting device 2 is moved over the skin 22 of the subject. Thus, the elongate elements 18 are generally arranged perpendicularly to the optical axis of the optical waveguide 4. The one or more elongate elements 18 are shaped and arranged with respect to each other to allow hair 22 on the skin 20 to reach and contact the optical waveguide 4 as the hair cutting device 2 is moved over the skin 20 of the subject.

As the guard elements 18 are intended to be moved over the skin 20, the side of the guard elements 18 that face the skin 20 can be shaped (e.g. profiled) so that they move or glide more easily over the skin.

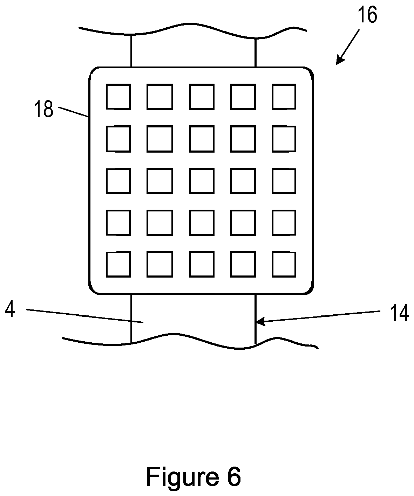

Fig. 6 is a bottom view of a guard structure 16 according to a second specific embodiment (i.e. a view of the skin-facing side of the guard structure 16 and hair cutting device 2). In the embodiment of Fig. 6, the guard structure 16 comprises a guard element 18 that is in the form of a grid or foil element that is arranged in the hair cutting device 2 so that when the hair cutting device 2 is in use, the guard element 18 is between the optical waveguide 4 and the skin 20. The guard element 18 comprises a plurality of holes that allow hairs to contact the optical waveguide 4. The holes can be regularly sized and/or spaced, or irregularly sized and/or spaced. In this embodiment, the guard element 18 can be similar to foils and grids used in conventional electric shavers.

In the illustrated embodiments, the optical waveguide 4 has a core 24. In the illustrated embodiments, the optical waveguide 4 does not include any cladding around the core 24. However it will be appreciated that in some embodiments the optical waveguide 4 can comprise cladding around the core 24, although preferably no cladding is present along the cutting face 14 (and indeed, in some embodiments the cutting face 14 can correspond to those parts of the optical waveguide 4 where there is no cladding).

The optical waveguide 4 is shown in contact with a hair 22 and the skin 20. The portion of the side wall of the core 16/optical waveguide 4 that is intended to contact hairs during use forms the cutting face 14. As described above, the refractive index of the core 24 is the same or lower than the refractive index of hair.

The core 24 may have a uniform refractive index (i.e. the same refractive index throughout the core 24), or it may be a graded index fibre, which means that the refractive index decreases with increasing distance from the optical axis.

It will be appreciated that although Fig. 4 (and the following figures) illustrate the one or more guard elements 18 as being generally flat or planar, in some embodiments the one or more guard elements 18 can be curved, for example they can be curved to follow the surface or shape of the optical waveguide 4.

As shown in Fig. 4, the one or more guard elements 18 are in contact with the optical waveguide 4. Due to this contact, it is possible for light in the optical waveguide 4 to couple into the one or more guard elements 18, which can lead to a loss of light energy/power (i.e. light coupling into the one or more guard elements 18 reduces the light energy/power available to couple into the hairs 22) and which can lead to the one or more guard elements 18 being heated.

Thus, in accordance with preferred embodiments of the invention, the one or more guard elements 18 in the guard structure 16 are shaped and/or formed to minimise or reduce the coupling of light from the optical waveguide 4 to the guard structure 16.

In some embodiments, since the amount of light that can couple into the one or more guard elements 18 depends on the size of the area of contact between the optical waveguide 4 and the one or more guard elements 18, the one or more guard elements 18 can be shaped so as to reduce or minimise the size of the area of contact between the optical waveguide 4 and the one or more guard elements 18.

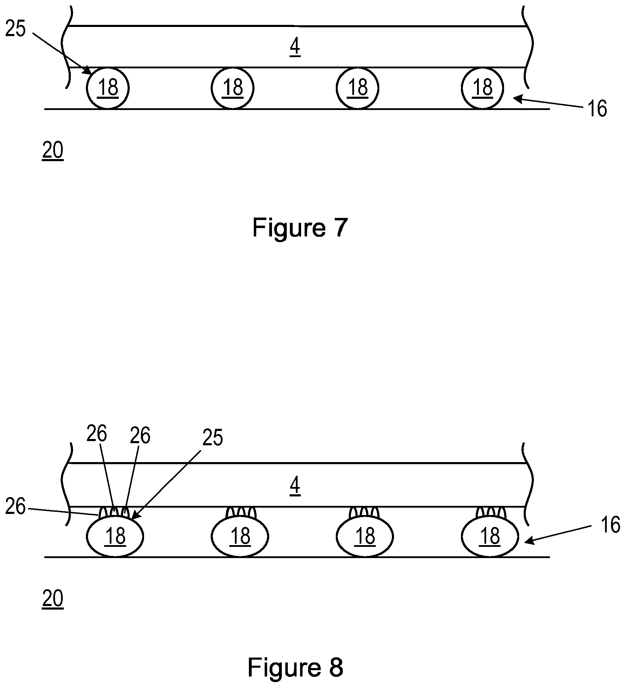

Fig. 7 illustrates an embodiment of a guard structure 16 in which the one or more guard elements 18 are shaped to minimise the area of contact, and in particular Fig. 7 shows a cross section through a guard structure 16 and an optical waveguide 4 (i.e. a view looking along the skin 20 towards the hair cutting device 2). It will be appreciated that the guard structure 16 shown in Fig. 7 can be according to the embodiment shown in Fig. 5 (i.e. elongate elements) or Fig. 6 (i.e. a grid or foil element). To minimise the area of contact, the side or surface of each guard element 18 (or the guard element 18 in the case of the embodiment in Fig. 6) that faces the optical waveguide 4 (which is referred to as upper surface 25) is curved or otherwise contoured. In the illustrated embodiment, the one or more guard elements 18 have a generally circular cross-section, but it will be appreciated that the one or more guard elements 18 can have a cross-section with a different regular or irregular shape, provided that the upper surface 25 facing the optical waveguide 4 is curved (i.e. the side or surface 25 is convex). Thus, compared to a guard element 18 with a flat upper surface 25, the area of contact between the one or more guard elements 18 and the optical waveguide 4 is significantly reduced when the upper surface 25 is curved.

Fig. 8 illustrates an alternative embodiment of a guard structure 16 in which the one or more guard elements 18 are shaped to minimise the area of contact. As with Fig. 7, Fig. 8 shows a cross section through a guard structure 16 and an optical waveguide 4. It will be appreciated that the guard structure 16 shown in Fig. 8 can be according to the embodiment shown in Fig. 5 (i.e. elongate elements) or Fig. 6 (i.e. a grid or foil element). In this embodiment the side or surface 25 of each guard element 18 that faces the optical waveguide 4 comprises one or more protrusions 26 that provide the contact between the one or more guard elements 18 and the optical waveguide 4. These protrusions 26 can be relatively small, for example a few tens or hundreds of micrometers or a few tens or hundreds of nanometers in width or diameter. The protrusions 26 can be patterned into or onto the upper surface 25 of the one or more guard elements 18, or they can be formed separately and attached to the one or more guard elements 18. Three protrusions 26 are shown for each guard element 18 in Fig. 8, but it will be appreciated that more or less protrusions 26 can be used as required. It will also be appreciated that Fig. 8 may not be drawn to scale, and that the protrusions 26 can have a different shape or cross-section to that shown in Fig. 8. Thus, compared to a guard element 18 with a flat upper surface 25, the area of contact with the optical waveguide 4 is significantly reduced when the upper surface 25 (which can be flat, curved or otherwise contoured) has one or more protrusions 26.

In some embodiments, which can be in addition to or alternative to those shown in Figs. 7 and 8, the coupling of light from the optical waveguide 4 to the guard structure 16 can be minimised or reduced by forming the guard structure 16, and specifically the one or more guard elements 18, from a material that has a lower refractive index than the optical waveguide 4. Suitable materials include Teflon, siliciumoxide, silver, aluminium and composites. Thus, due to the one or more guard elements 18 being formed from a material with a lower refractive index than the optical waveguide 4, light in the optical waveguide 4 will not refract into the one or more guard elements 18.

The one or more guard elements 18 can be completely formed from the material that has a lower refractive index than the optical waveguide 4. However, in other embodiments only a portion of the one or more guard elements 18 (in particular including the portion that contacts the optical waveguide 4) is formed from a material that has a lower refractive index than the optical waveguide 4. The portion of the one or more guard elements 18 can be a layer or coating of the material on the upper surface 25 of the one or more guard elements 18. Suitable materials for the layer or coating include Teflon, siliciumoxide, silver, aluminum, composites, or dielectric materials.

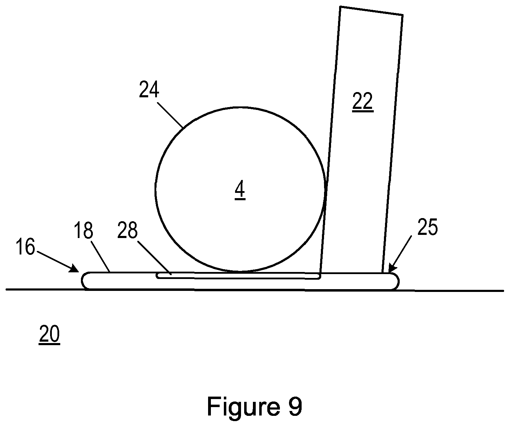

In some embodiments, which can be in addition to or alternative to any of those described above, the guard elements 18, or a portion of the guard elements 18 that face the optical waveguide 4, can be formed with a coating or layer of an optically reflective material. Suitable optically reflective materials include silver or aluminium. This embodiment is illustrated in Fig. 9 which shows a portion 28 on the guard element 18 below the optical waveguide 4. Thus, the reflective material acts to reflect light from the optical waveguide 4 so that it does not heat the guard element 18. It will be appreciated that in alternative embodiments the whole of the upper surface 25 can be formed with the coating or layer of reflective material. In other alternative embodiments, the guard elements 18 can be completely formed from the optically reflective material.

It will be appreciated that in the case of wet shaving, some form of fluid (e.g. shaving foam or gel) is used, so it is desirable to prevent or reduce the build-up of fluid between the guard elements 18 and between the guard elements 18 and the optical waveguide 4. Therefore, in some embodiments, the guard elements 18 can be coated with or comprise a hydrophobic and/or oleophobic layer (with the type of material depending on the type of fluid to be used (e.g. whether it is a water or oil-based fluid). Suitable materials include nano-coatings, for example based on fluorinated compounds. In some embodiments, the layer can be formed from a superhydrophobic material, for example a patterned rare-earth ceramic.

Although in the above embodiments the guard elements 18 are in contact with the optical waveguide 4, it will be appreciated that the guard elements 18, and more generally the guard structure 16, may not be held in place in the hair cutting device 2 by this contact, particularly since the optical waveguide 4 can be a relatively fragile part of the hair cutting device 2. Instead, as well as the contact between the optical waveguide 4 and the guard structure 16, the guard structure 16 can be connected to another part of the hair cutting device 2, for example to another part of the head portion 12 in the embodiment shown in Fig. 2. This connection can be a rigid connection or a flexible connection, with the latter option being advantageous for allowing the guard structure 16 to follow the contours of the skin.

Therefore the guard structure 16 described above reduces the risk of causing damage or injury to the skin of the subject when used in a hair cutting device that uses light to cut or burn the hair.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A hair cutting device (2) for cutting hair on a body of a subject, the hair cutting device (2) comprising:
a light source (6) for generating laser light at one or more specific wavelengths corresponding to wavelengths absorbed by one or more chromophores in hair;
a cutting element that comprises an optical waveguide (4) that is coupled to the light source (6) to receive laser light and
a guard structure (16) that comprises:
one or more guard elements (18), wherein the one or more guard elements (18) are arranged such that in use the one or more guard elements (18) space the optical waveguide (4) from the skin (20) of the subject, **characterized in that**
a portion of a sidewall of the optical waveguide forms a cutting face for contacting hair; and **in that**
the one or more guard elements (18) are shaped and/or formed to minimise or reduce the coupling of light from the optical waveguide (4) to the guard structure (16).

2. The hair cutting device of claim 1, wherein the one or more guard elements (18) are formed from a material having a lower refractive index than the optical waveguide (4).

3. The hair cutting device of claim 1 or 2, wherein the one or more guard elements (18) are formed with a layer or coating of material that has a lower refractive index than the optical waveguide (4).

4. The hair cutting device of any of claims 1-3, wherein the one or more guard elements (18) are shaped to reduce or minimise the contact area between the guard elements (18) and the optical waveguide (4).

5. The hair cutting device of any of claims 1-4, wherein a side (25) of each of said one or more guard elements (18) that is in contact with the optical waveguide (4) is curved.

6. The hair cutting device of any of claims 1-5, wherein a side (25) of each of said one or more guard elements (18) that is in contact with the optical waveguide (4) comprises one or more protrusions (26) that provide the contact between the one or more guard elements (18) and the optical waveguide (4).

7. The hair cutting device of any of claims 1-6, wherein the one or more guard elements (18) comprise a coating or layer of an optically reflective material on at least a side (25) of the one or more guard elements (18) that contact the optical waveguide (4).

8. The hair cutting device of any of claims 1-7, wherein the one or more guard elements (18) are coated with or comprise a hydrophobic and/or oleophobic layer.

9. The hair cutting device of any of claims 1-8, wherein the one or more guard elements (18) are elongate elements.

10. The hair cutting device of any of claims 1-8, wherein the one or more guard elements (18) is a grid or foil.

## Patentansprüche

1. Haarschneidevorrichtung (2) zum Schneiden von Haaren auf einem Körper eines Subjekts, wobei die Haarschneidevorrichtung (2) umfasst:
eine Lichtquelle (6) zum Erzeugen von Laserlicht in einer oder mehreren spezifischen Wellenlängen, die Wellenlängen entsprechen, die von einem oder mehreren Chromophoren im Haar absorbiert werden;
ein Schneideelement, das einen optischen Wellenleiter (4) umfasst, der mit der Lichtquelle (6) gekoppelt ist, um Laserlicht zu empfangen und
eine Schutzstruktur (16), die umfasst:
eines oder mehrere Schutzelemente (18), wobei das eine oder die mehreren Schutzelemente (18) so angeordnet sind, dass in Verwendung das eine oder die mehreren Schutzelemente (18) den optischen Wellenleiter (4) von der Haut (20) des Subjekts in Abstand halten, **dadurch gekennzeichnet, dass**
ein Abschnitt einer Seitenwand des optischen Wellenleiters eine Schneidefläche bildet, um mit dem Haar in Berührung zu kommen; und dadurch, dass
das eine oder die mehreren Schutzelemente (18) gestaltet und/oder gebildet sind, um das Koppeln von Licht von dem optischen Wellenleiter (4) mit der Schutzstruktur (16) zu minimieren oder zu reduzieren.

2. Haarschneidevorrichtung nach Anspruch 1, wobei das eine oder die mehreren Schutzelemente (18) aus einem Material gebildet sind, das einen niedrigeren Brechungsindex aufweist als der optische Wellenleiter (4).

3. Haarschneidevorrichtung nach Anspruch 1 oder 2, wobei das eine oder die mehreren Schutzelemente (18) mit einer Schicht oder Beschichtung aus Material gebildet ist, das einen niedrigeren Brechungsindex aufweist als der optische Wellenleiter (4).

4. Haarschneidevorrichtung nach einem der Ansprüche 1-3, wobei das eine oder die mehreren Schutzelemente (18) gestaltet sind, um die Kontaktfläche zwischen den Schutzelementen (18) und dem optischen Wellenleiter (4) zu reduzieren oder zu minimieren.

5. Haarschneidevorrichtung nach einem der Ansprüche 1-4, wobei eine Seite (25) von jedem des einen oder der mehreren Schutzelemente (18), die mit dem optischen Wellenleiter (4) in Berührung kommt, gekrümmt ist.

6. Haarschneidevorrichtung nach einem der Ansprüche 1-5, wobei eine Seite (25) von jedem des einen oder der mehreren Schutzelemente (18), die mit dem optischen Wellenleiter (4) in Berührung kommt, einen oder mehrere Vorsprünge (26) umfasst, die den Kontakt zwischen dem einen oder den mehreren Schutzelementen (18) und dem optischen Wellenleiter (4) bereitstellen.

7. Haarschneidevorrichtung nach einem der Ansprüche 1-6, wobei das eine oder die mehreren Schutzelemente (18) eine Beschichtung oder Lage aus einem optisch reflektierenden Material an mindestens einer Seite (25) des einen oder der mehreren Schutzelemente (18), die mit dem optischen Wellenleiter (4) in Berührung kommen, umfassen.

8. Haarschneidevorrichtung nach einem der Ansprüche 1-7, wobei das eine oder die mehreren Schutzelemente (18) mit einer wasserabweisenden und/oder ölabweisenden Schicht beschichtet sind oder umfassen.

9. Haarschneidevorrichtung nach einem der Ansprüche 1-8, wobei das eine oder die mehreren Schutzelemente (18) längliche Elemente sind.

10. Haarschneidevorrichtung nach einem der Ansprüche 1-8, wobei das eine oder die mehreren Schutzelemente (18) ein Gitternetz oder eine Folie sind.

## Revendications

1. Dispositif de coupe de poils (2) pour couper les poils sur le corps d'un sujet, le dispositif de coupe de poils (2) comprenant :
une source de lumière (6) pour générer une lumière laser à une ou plusieurs longueurs d'onde particulières correspondant aux longueurs d'ondes absorbées par un ou plusieurs chromophores dans les poils ;
un élément de coupe qui comprend un guide d'onde optique (4) qui est couplé à la source de lumière (6) pour recevoir la lumière laser et
une structure de protection (16) qui comprend :
un ou plusieurs éléments de protection (18), dans laquelle le ou les éléments de protection (18) sont agencés de sorte que lors de l'utilisation le ou les éléments de protection (18) espacent le guide d'onde optique (4) de la peau (20) du sujet, **caractérisé en ce que**
une partie d'une paroi latérale du guide d'onde optique forme une face de coupe pour venir en contact avec les poils ; et **en ce que**
le ou les éléments de protection (18) sont façonnés et/ou formés pour minimiser ou réduire le couplage de la lumière du guide d'onde optique (4) à la structure de protection (16).

2. Dispositif de coupe de poils selon la revendication 1, dans lequel le ou les éléments de protection (18) sont formés à partir d'un matériau présentant un indice de réfraction plus faible que celui du guide d'onde optique (4).

3. Dispositif de coupe de poils selon la revendication 1 ou 2, dans lequel le ou les éléments de protection (18) sont formés avec une couche ou un revêtement de matériau qui présente un indice de réfraction plus faible que celui du guide d'onde optique (4).

4. Dispositif de coupe de poils selon l'une quelconque des revendications 1 à 3, dans lequel le ou les éléments de protection (18) sont façonnés pour réduire ou minimiser la zone de contact entre les éléments de protection (18) et le guide d'onde optique (4).

5. Dispositif de coupe de poils selon l'une quelconque des revendications 1 à 4, dans lequel un côté (25) de chacun dudit ou desdits éléments de protection (18) qui est en contact avec le guide d'onde optique (4) est incurvé.

6. Dispositif de coupe de poils selon l'une quelconque des revendications 1 à 5, dans lequel un côté (25) de chacun dudit ou desdits éléments de protection (18) qui est en contact avec le guide d'onde optique (4) comprend une ou plusieurs saillies (26) qui assurent le contact entre le ou les éléments de protection (18) et le guide d'onde optique (4).

7. Dispositif de coupe de poils selon l'une quelconque des revendications 1 à 6, dans lequel le ou les éléments de protection (18) comprend un revêtement ou une couche d'un matériau optique réfléchissant sur au moins un côté (25) de l'un ou plusieurs des éléments de protection (18) qui entre en contact avec le guide d'onde optique (4).

8. Dispositif de coupe de poils selon l'une quelconque des revendications 1 à 7, dans lequel le ou les éléments de protection (18) sont recouverts avec ou comprennent une couche hydrophobe et/ou oléophobe.

9. Dispositif de coupe de poils selon l'une quelconque des revendications 1 à 8, dans lequel le ou les éléments de protection (18) sont des éléments allongés.

10. Dispositif de coupe de poils selon l'une quelconque des revendications 1 à 8, dans lequel le ou les éléments de protection (18) sont une grille ou une feuille.
